# EUROPEAN PATENT APPLICATION

(11) **EP 1 076 064 A1**
(43) Date of publication of application: **14.02.2001**
(21) Application number: 99917152.3
(22) Date of filing: 26.04.1999
(51) Int. Cl.: C07K 5/075, C07K 1/02

(54) **NOVEL ASPARTAME DERIVATIVE CRYSTAL AND PROCESS FOR PRODUCING THE SAME**

(30) Priority: 08.05.1998 JP 12599298
(71) Applicant: Ajinomoto Co., Inc., Tokyo 104-8315 (JP)
(72) Inventor: KISHISHITA, Akihiro, Ajinomoto Co., Inc., Kawasaki-shi, Kanagawa 210-0801 (JP); NAGASHIMA, Kazutaka, Ajinomoto Co., Inc., Kawasaki-shi, Kanagawa 210-0801 (JP); ISHIDA, Hirotoshi, Ajinomoto Co., Inc., Kawasaki-shi, Kanagawa 210-0801 (JP); NAGAI, Takeshi, Ajinomoto Co., Inc., Kawasaki-shi, Kanagawa 210-0801 (JP)
(74) Representative: Nash, David Allan
(86) International application number: JP9902200
(87) International publication number: WO9958554

(57) **Abstract**

In precipitation of N-[N-(3,3-dimethylbutyl)-L-α -aspartyl]-L-phenylalanine methyl ester from an aqueous solution of said ester, its crystal is precipitated at a crystallization temperature of 25 °C or lower and the solution is further cooled, if necessary or if desired, and the precipitated crystal (B-type crystal) is separated by solid-liquid separation and then dried.

The crystal thus dried is a novel crystal (D-type crystal) of said ester excellent in dissolution rate, and this crystal when measured by powder X-ray diffractometry, shows characteristic peaks in X-ray diffraction pattern at diffraction angles (2θ, CuKα radiation) of at least 5.4°, 8.4°, 18.8° and 17.6°.

## Description

### TECHNICAL FIELD

The present invention relates to a novel crystal of high-potency sweetener N-[N-(3,3-dimethylbutyl)-L-α-aspartyl]-L-phenylalanine methyl ester with improved dissolution characteristics (solubility), and also to a process for producing the same. For reference's sake, as is well-known, L-α-aspartyl-L-phenylalanine methyl ester (APM) is a kind of amino acid-based high-potency (highly sweet) sweetener whose commercialization has already been established, and it is abbreviated to "APM" or "Aspartame". Accordingly, said sweetener according to the present invention can be regarded as a derivative of "APM" or "Aspartame", and therefore is abbreviated hereinafter to "N-(3,3-dimethylbutyl)-APM".

### BACKGROUND ART

The efficacy of sweetness of N-(3,3-dimethylbutyl)-APM is at least 50 times that of Aspartame in weight ratio and is about 10,000 times that of sucrose (table sugar), and thus it can constitute a very strong sweetener.

Because sweeteners are mainly intended for use in foods and consumed by persons, they should be prepared by processes enabling preparation of high-purity products substantially free from impurities or decomposed materials. To be usable in an industrial scale, such processes should have been established so as to be reproducible at relatively low costs.

The crystal structure of already known N-(3,3-dimethylbutyl)-APM is described as IR spectrum data in WO95/30689. Further, the present inventors analyzed the structure of its single crystal, and as a result, they confirmed that this crystal is a monohydrate, and when measured by powder X-ray diffractometry, the crystal shows characteristic peaks in diffractive X-ray (X-ray diffraction pattern) at diffraction angles (angle of diffraction) of at least 6.0°, 24.8°, 8.2°, and 16.5° (2 θ, CuKα radiation (ray; line)). For the sake of convenience, the present inventors referred to this crystal as "A-type crystal".

However, the dissolution rate of the A-type crystal in water is low, so there is a commercial and industrial problem on the product qualities.

Under the background of such prior art, the object of (and problem to be solved by) the present invention is to provide a novel crystal of a high-potency sweetener N-(3,3-dimethylbutyl)-APM with improved dissolution rate (solubility).

### DISCLOSURE OF INVENTION

As a result of their eager study to resolve the above-described problem and achieve the object, the present inventors have found that the temperature at which precipitation of crystals occurs (crystallization temperature) is controlled in the course of crystallization from an aqueous solution containing N-(3,3-dimethylbutyl)-APM, and the crystals precipitated in this manner are subjected to solid/liquid separation and then dried whereby a novel crystal of N-(3,3-dimethylbutyl)-APM with improved dissolution characteristics can be obtained, and on the basis of such findings, the present invention has been completed. For reference's sake, this novel crystal is hereinafter referred to as "D-type crystal".

That is, the present invention relates to a novel crystal (D-type crystal) of N-(3,3-dimethylbutyl)-APM which when measured by powder X-ray diffractometry using CuKα radiation (ray), shows characteristic peaks in diffractive X-ray (X-ray diffraction pattern) at diffraction angles other than those of A-type crystal, that is, at least 5.4°, 8.4°, 18.8°, and 17.6° (2 θ, CuKα radiation), as well as the process for producing said crystal.

To obtain the D-type crystal of the present invention, e.g. the following procedures can be used. For example, when an aqueous solution of N-(3,3-dimethylbutyl)-APM is subjected to crystallization, the crystallization temperature is controlled in the range of 25 °C or less, preferably 20 °C or less, more preferably 10 °C or less, and the precipitated crystals are subjected to solid/liquid separation and dried whereby the objective D-type crystals can be obtained.

Many kinds of processes, such as various synthesis processes (methods) are known for the process for producing N-(3,3-dimethylbutyl)-APM, but it goes without saying that the crystal of the present invention can be obtained by not depending on the kinds of processes for producing N-(3,3-dimethylbutyl)-APM itself.

As a crystallization solvent for N-(3,3-dimethylbutyl)-APM, an aqueous solvent (water alone or a mixture of water and an organic solvent such as ethyl alcohol, methyl alcohol, acetonitrile, etc. at an arbitrary ratio) can be used, but water is preferably used.

The crystallization point (crystallization temperature) can be controlled very easily by those skilled in the art by controlling the concentration of N-(3,3-dimethylbutyl)-APM before crystallization, cooling rate, stirring rate, and etc.

It goes without saying that after the crystals are precipitated, the slurry is cooled further to lower temperature and stirred whereby a higher yield of crystals can be secured.

The crystals precipitated in the crystallization are subjected to solid-liquid separation. The wet crystals thus obtained show characteristic peaks at diffraction angles of at least 5.1°, 21.1°, 21.3°, and 8.3° (2 θ, CuKα radiation) in powder X-ray diffractometry. For the sake of convenience, these crystals are referred to as "B-type crystals". The solid-liquid separation methods include, but are not limited to, filtration, centrifugation, and etc.

The B-type crystals are simply dried whereby the objective crystals of N-(3,3-dimethylbutyl)-APM can be produced.

A dryer (drying equipment) for drying B-type crystals to convert them into D-type crystals is not limited. Although a through-flow dryer, a fluidized bed dryer, a vacuum dryer, a spray-dryer, a micron dryer, and etc. can be used, a vacuum dryer is preferably used.

The degree of drying B-type crystals to convert them into D-type crystals is preferably until their water content is reduced to 3 to 6 % by weight.

The crystals thus obtained may be used directly as the product, or may be subjected if necessary or if desired to granulation to give the product having a larger particle size. The granulator (granulating machine) in this case is not particularly limited, but a roll press granulator (compactor) is preferable.

### BEST MODE FOR CARRYING OUT THE INVENTION

Hereinafter, the present invention is described in more detail with making reference to Reference Examples and Examples.

### Reference Example 1: Preparation of N-(3,3-dimethylbutyl)-APM

The following were introduced successively under stirring to a reactor equipped with an agitating blade for ensuring very efficient transfer of gaseous hydrogen to a liquid layer (solution). That is, 700 ml of ion exchanged water, 4.21 ml of acetic acid, 20 g of 10 % palladium carbon, 1,300 ml of methanol, 56 g of Aspartame and 25 ml of 3,3-dimethylbutylaldehyde were introduced thereinto.

The reactor was filled with a nitrogen gas stream, and then the reaction mixture was hydrogenated at a H₂ gas flow rate of 200 ml/min at room temperature. The progress of this reaction was monitored by sampling the reaction mixture and analyzing the product in high performance liquid chromatography (HPLC). After the hydrogenation reaction for 6 hours, this reaction was terminated by filling the reactor with a nitrogen gas stream and filtering the reaction mixture through a fine pore filter (0.45 µm) to remove the catalyst.

As a result of the analysis of thus obtained filtrate (1,494 g), the yield was 81%. Subsequently, this filtrate was concentrated to 281 g to remove the methanol, and crystals were precipitated by stirring at 10 °C overnight. Finally, 87 g white wet crystals of N-(3,3-dimethylbutyl)-APM (yield: 77 %) were obtained at a high purity (99 % or higher, HPLC).

### Reference Example 2: Production of B-type crystals (No. 1)

Part of N-(3,3-dimethylbutyl)-APM prepared in Reference Example 1 was used to prepare 100 g aqueous solution of N-(3,3-dimethylbutyl)-APM at a concentration of 2.5 % by weight (dissolved at 60 °C). Then, the solution was cooled from 60°C to 25°C over 5 minutes under stirring. When the liquid temperature reached to 25 °C, crystallization of white crystals was initiated and found. The liquid temperature was reduced to 20 °C, and after overnight aging, the crystals were collected by filtration.

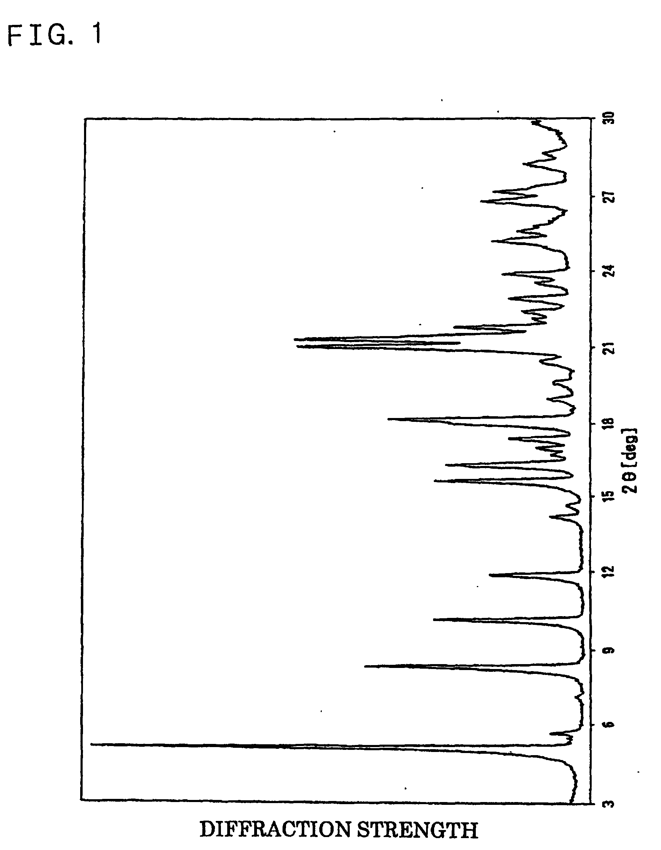

The diffractive X-ray (X-ray diffraction pattern) of the wet crystals obtained above was measured by powder X-ray diffractometry (diffractometer) using CuKα radiation (ray). The obtained powder X-ray diffraction pattern is shown in Fig. 1.

As is evident from the pattern of the figure, the wet crystals showed characteristic diffraction peaks at least at 5.1°, 21.1°, 21.3°, and 8.3°. As mentioned above, the crystals are B-type crystals.

### Example 1: Production of D-type crystals (No. 1)

The above wet B-type crystals were dried in a vacuum tray (shelves) dryer at 50 °C until their water content was reduced to 5 % by weight.

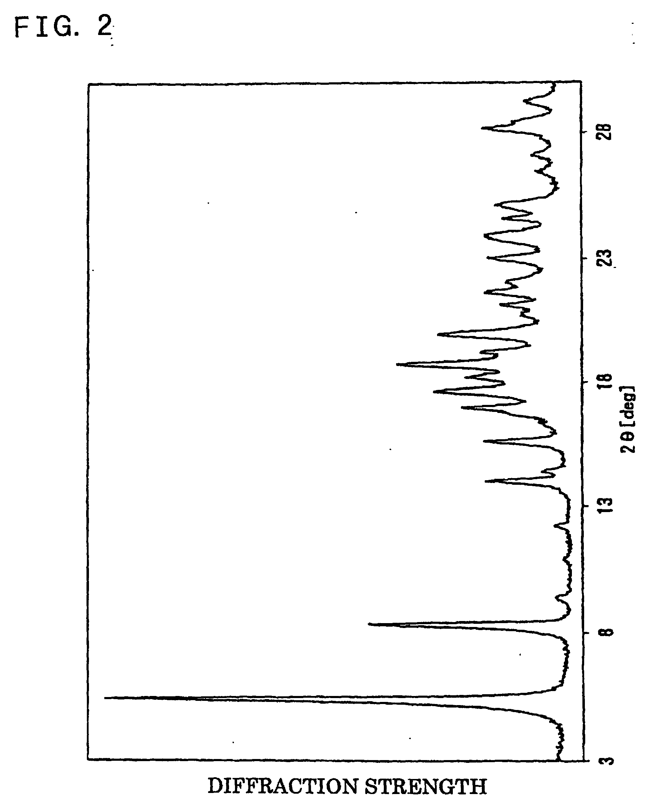

The diffractive X-ray (X-ray diffraction pattern) of the dried crystals was measured by powder X-ray diffractometry (diffractometer) using CuKα radiation. The obtained powder X-ray diffraction pattern is shown in Fig. 2.

As is evident from the pattern of the figure, the dried crystals showed characteristic diffraction peaks at least at 5.4°, 8.4°, 18.8°, and 17.6°. As described above, the crystals are D-type crystals.

### Reference Example 3: Production of B-type crystals (No. 2)

Part of N-(3,3-dimethylbutyl)-APM prepared in Reference Example 1 was used to prepare 100 g aqueous solution of N-(3,3-dimethylbutyl)-APM at a concentration of 2 % by weight (dissolved at 60 °C). Then, the solution was cooled from 60°C to 10°C over 5 minutes under stirring. When the liquid temperature reached to 10 °C, crystallization of white crystals was initiated. After overnight aging with the liquid temperature kept at 10 °C, the crystals were collected by filtration.

The X-ray diffraction pattern of the wet crystals obtained above was measured by powder X-ray diffractometry using CuKα radiation, and as a result, the crystals were identified as B-type crystals.

### Example 2: Production of D-type crystals (No. 2)

The above wet B-type crystals were dried in a vacuum tray dryer at 50 °C until their water content was reduced to 5 % by weight.

The X-ray diffraction pattern of the dried crystals was measured by powder X-ray diffractometry using CuKα radiation, and as a result, the crystals were identified as D-type crystals.

### Reference Example 4: Production of A-type crystals

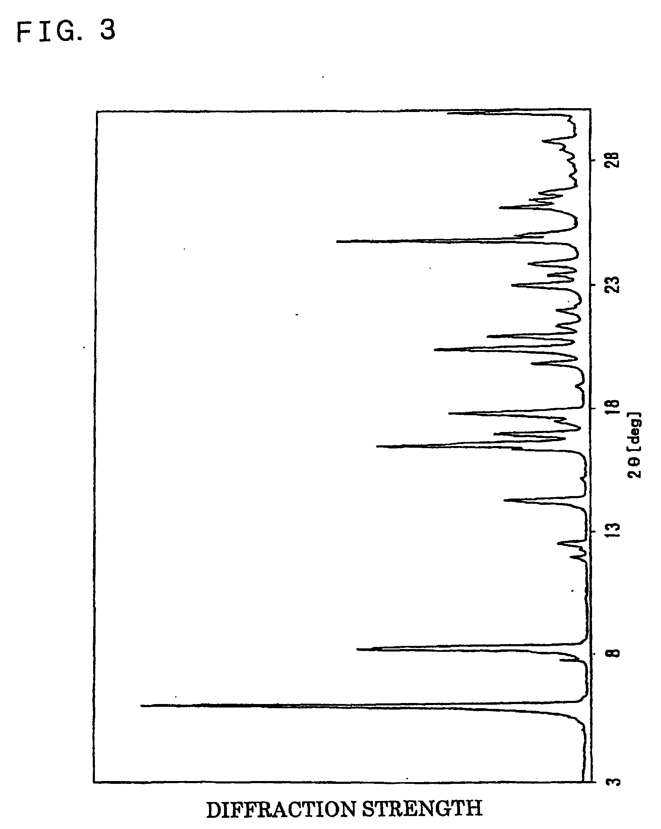

N-(3,3-dimethylbutyl)-APM prepared in Reference Example 1 was used to prepare 100 g aqueous solution of N-(3,3-dimethylbutyl)-APM at a concentration of 3 % by weight (dissolved at 60°C). Then, the solution was cooled from 60 °C to 30 °C over 5 minutes under stirring. When the liquid temperature reached to 30 °C, crystallization of white crystals was initiated. After overnight aging under the temperature kept at 30 °C for the liquid, the crystals were collected by filtration.
(a) The X-ray diffraction pattern of the wet crystals obtained above was measured by powder X-ray diffractometry using CuKα radiation. The obtained powder X-ray diffraction pattern is shown in Fig. 3.

As is evident from the pattern of the figure, the wet crystals showed characteristic diffraction peaks for the A-type crystal at least at 6.0°, 24.8°, 8.2° and 16.5°.

Further, (b) the wet crystals were dried in a vacuum tray dryer set at 50 °C, to give dried crystals with a water content of 5% by weight. The dried crystals were measured by powder X-ray diffractometry using CuKα radiation, indicating that the crystals also were A-type crystals as well.

Further, as a result of IR spectrum (KBr) measurement, its values agreed with those described in WO95/30689.

### Test Example 1: Measurement of the dissolution rate for the dried crystals

The dissolution rates of the A-type crystals (Reference Example 4 (b)) and the D-type crystals (Example 1) were determined in the following method. The apparatus used was "DISSOLUTION TESTER" (NTR-6100) manufactured by Toyama Sangyo K. K. 0.5 g each of the A-type crystals and D-type crystals were introduced into 900 ml ion exchanged water kept at 20 °C at a stirring in the number of revolution of 100 rpm, and the time which elapsed until the crystals came to be completely dissolved was measured.

The result indicated that the dissolution of the A-type crystals required about 35 minutes, whereas the D-type crystals were dissolved completely in only about 5 minutes.

As is evident from the foregoing, the D-type crystals of the present invention were found to be useful crystals with significantly improved dissolution characteristics, as compared with the A-type crystals.

### BRIEF DESCRIPTION OF DRAWINGS

FIG. 1: A powder X-ray diffraction pattern of B-type crystals.
FIG. 2: A powder X-ray diffraction pattern of D-type crystals.
FIG. 3: A powder X-ray diffraction pattern of A-type crystals.

### INDUSTRIAL APPLICABILITY

A novel crystal of high-potency sweetener N-(3,3-dimethylbutyl)-APM with improved dissolution characteristics can be provided and also can be used as a table sweetener, a sweetener for producing drinks, and a sweetener for the others.

## Claims

1. A novel crystal of N-[N-(3,3-dimethylbutyl)-L-α-aspartyl]-L-phenylalanine methyl ester showing characteristic peaks in X-ray diffraction pattern at diffraction angles (2θ, CuKα radiation) of at least 5.4°, 8.4°, 18.8° and 17.6°.

2. A process for producing the novel crystal according to claim 1, which comprises:
in precipitation of N-[N-(3,3-dimethylbutyl)-L-α-aspartyl]-L-phenylalanine methyl ester from an aqueous solution of N-[N-(3,3-dimethylbutyl)-L-α-aspartyl]-L-phenylalanine methyl ester, precipitating a crystal thereof at a crystallization temperature of 25 °C or lower and further cooling the solution, if necessary or if desired, separating the precipitated crystal by solid-liquid separation, and then drying the crystal.

3. The process for producing the novel crystal according to claim 2, which comprises drying said precipitated crystal until its water content is reduced to 3 to 6 % by weight.
